# EUROPEAN PATENT SPECIFICATION

(11) **EP 3 884 054 B1**
(45) Date of publication and mention of the grant of the patent: **11.11.2026**
(21) Application number: 19806232.5
(22) Date of filing: 21.11.2019
(51) Int. Cl.: C12N 15/81, C12R 1/72, C12P 19/44, C12N 1/16

(54) **IMPROVED PRODUCTION OF SYMMETRICAL BOLAFORM SOPHOROSIDES**
VERBESSERTE HERSTELLUNG VON SYMMETRISCHEN BOLAFORM-SOPHOROSIDEN
PRODUCTION AMÉLIORÉE DE SOPHOROSIDES BOLAFORMES SYMÉTRIQUES

(30) Priority: 22.11.2018 EP 18207793
(43) Date of publication of application: 29.09.2021
(73) Proprietor: Universiteit Gent, 9000 Gent (BE); Amphistar, 9052 Gent (BE)
(72) Inventor: ROELANTS, Sophie, 9090 Melle (BE); VAN RENTERGHEM, Lisa, 9940 Evergem (BE); SOETAERT, Wim, 9920 Lievegem (BE); REMMERY, Jelle, 9000 Gent (BE)
(74) Representative: De Clercq & Partners
(86) International application number: PCT/EP2019/082063
(87) International publication number: WO 2020/104582

(56) References cited:
- WO-A1-2011/154523
- WO-A1-2015/028278
- INGE N. A. VAN BOGAERT ET AL: "The biosynthetic gene cluster for sophorolipids: a biotechnological interesting biosurfactant produced by Starmerella bombicola", MOLECULAR MICROBIOLOGY, vol. 88, no. 3, 21 March 2013 (2013-03-21), pages 501 - 509, XP055146054, ISSN: 0950-382X, DOI: 10.1111/mmi.12200
- LISA VAN RENTERGHEM ET AL: "From lab to market: An integrated bioprocess design approach for new-to-nature biosurfactants produced by Starmerella bombicola", BIOTECHNOLOGY AND BIOENGINEERING, vol. 115, no. 5, 4 February 2018 (2018-02-04), pages 1195 - 1206, XP055569212, ISSN: 0006-3592, DOI: 10.1002/bit.26539
- LODENS SOFIE ET AL: "Transformation of an Exotic Yeast Species into a Platform Organism: A Case Study for Engineering Glycolipid Production in the Yeast Starmerella bombicola", 1 January 2018, SYNTHETIC BIOLOGY : METHODS AND PROTOCOLS; IN: METHODS IN MOLECULAR BIOLOGY; ISSN 1064-3745 ; VOL. 1772; [METHODS IN MOLECULAR BIOLOGY; ISSN 1064-3745 ; VOL. 1772], HUMANA PRESS, US, PAGE(S) 95 - 123, ISBN: 978-1-4939-7794-9, XP009511886
- ROBIN GEYS ET AL: "Increasing Uniformity of Biosurfactant Production in Starmerella bombicola via the Expression of Chimeric Cytochrome P450s", COLLOIDS AND INTERFACES, vol. 2, no. 4, 3 October 2018 (2018-10-03), pages 42, XP055569265, DOI: 10.3390/colloids2040042
- TAKAHASHI FUMIKAZU ET AL: "Identification of the fatty alcohol oxidase FAO1 fromStarmerella bombicolaand improved novel glycolipids production in anFAO1knockout mutant", APPLIED MICROBIOLOGY AND BIOTECHNOLOGY, SPRINGER, DE, vol. 100, no. 22, 6 July 2016 (2016-07-06), pages 9519 - 9528, XP036081175, ISSN: 0175-7598, [retrieved on 20160706], DOI: 10.1007/S00253-016-7702-6
- INGE N A VAN BOGAERT ET AL: "Microbial synthesis of sophorolipids", PROCESS BIOCHEMISTRY, ELSEVIER LTD, GB, vol. 46, no. 4, 11 January 2011 (2011-01-11), pages 821 - 833, XP028169065, ISSN: 1359-5113, [retrieved on 20110118], DOI: 10.1016/J.PROCBIO.2011.01.010
- SOPHIE L.K.W ROELANTS ET AL: "Candida bombicola as a platform organism for the production of tailor-made biomolecules", BIOTECHNOLOGY AND BIOENGINEERING, 1 September 2013 (2013-09-01), United States, pages 2494 - 2503, XP055569207, Retrieved from the Internet <URL:doi:10.1002/bit.24895> DOI: 10.1002/bit.24895

## Description

### Technical field of invention

The present invention relates to the field of microbial production of novel biosurfactants. More specifically, the present invention discloses the usage of a fungal strain such as the yeast *Starmerella bombicola* having a non-functional CYP52M1 cytochrome P450 monooxygenase and a non-functional FAO1 fatty alcohol oxidase, wherein the fungal strain is a yeast selected from the group consisting of *Starmerella (Candida) bombicola, Candida apicola, Candida batistae, Candida floricola, Candida riodocensis, Candida stellate, Candida kuoi, Candida sp. NRRL Y-27208, Rhodotorula bogoriensis sp., Wickerhamiella domericqiae* and a sophorolipid-producing strain of the *Starmerella* clade, for producing high amounts of so-called "symmetrical (acetylated) bolaform sophorosides" where both sophorose moieties are connected to the hydrophobic linker by a glycosidic bound. In addition, the present invention further discloses that the latter yeast can also be used to produce (acetylated) alkyl sophorosides.

### Background art

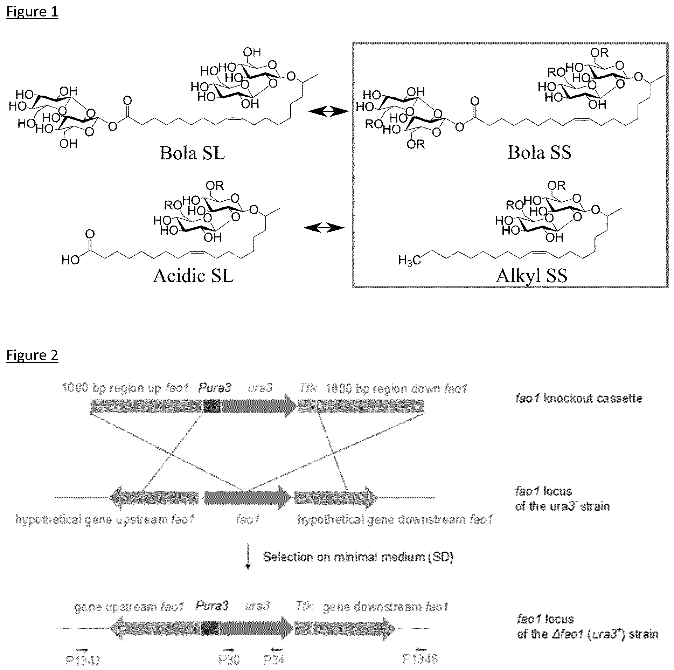

It has been demonstrated that the industrial sophorolipid producing yeast *Starmerella bombicola* can be exploited as a platform organism for the production of glycolipids and other biochemicals (Roelants *et al.,* 2013; Roelants *et al.,* 2016). One of the most recent achievements in this respect, describes the redesigning of this yeast to produce so-called bola sophorosides (SSs) (Van Renterghem *et al.,* 2018) (Figure 1). These bola SSs are a novel type of glycolipid which provide a more chemically stable alternative to the earlier described bola sophorolipids (SLs) (Soetaert *et al.,* 2013; Van Bogaert *et al.,* 2016). Indeed, Van Renterghem *et al.* (2018) disclose that a *S. bombicola* strain deficient in its acetyltransferase (*at*), lactonase esterase (*sble*) and alcohol oxidase 1 (*foo1*) when fed with fatty alcohols produces bola-sophorosides. However, the latter bola-sophorosides comprise -besides bola-sophorosides- still substantial amounts of bolaform sophorolipids. There is thus still a need for further strain improvements in order to shift the bolaform sophorolipid production more towards the sophoroside compounds. Moreover, the bola sophorosides produced by Van Renterghem are a mixture of non-symmetrical i.e. one sophorose moiety is subterminally linked, while the other one is terminally linked and symmetrical bola sophorosides (both sophoroses are terminally linked). The molecules described by Van Renterghem are non-acetylated due to the deletion of the *at* gene (acetyltransferase).

Takahashi et al. (2016) described the increased production of alkyl sophorosides by a *S. bombicola* strain having a deleted *fao1* gene as compared to the wild type fed with fatty alcohols. However, this increase in production is still accompanied with production of acidic and lactonic sophorolipids, which is undesired.

Van Bogaert et al. (2013) identified the cytochrome P450 monooxygenase CYP52M1 as the first enzyme of the sophorolipid biosynthetic pathway in *S. bombicola.* Knocking-out the CYP52M1 gene in *S. bombicola* resulted in abolishment of sophorolipid production (Van Bogaert et al. (2013), WO 2011/154523 A1 and Lodens et al. (2018)).

### Brief description of figures

Figure 1: Chemical structures of sophorolipids (SLs) (left) and sophorosides (SSs) (right, circled in grey). Bola SLs and acidic SLs are presented as a comparison. Glycosidic and ester linkages are indicated to highlight the difference between bola sophorolipids and sophorosides, whereas the methyl group of alkyl SS is indicated to highlight the difference between the acidic SLs having a carboxylic group. R groups represents either an H or acetyl (COCH3) group.
Figure 2: Schematic representation of the fatty alcohol oxidase (*fao1*) knockout cassette and integration thereof at the *fao1* locus of the *ura3* auxotrophic *Δcyp52M1* strain. The *ura3* gene was used as marker gene. The respective primer pairs for colony PCR to check correct integration of the *fao1* knockout cassette are depicted in orange.

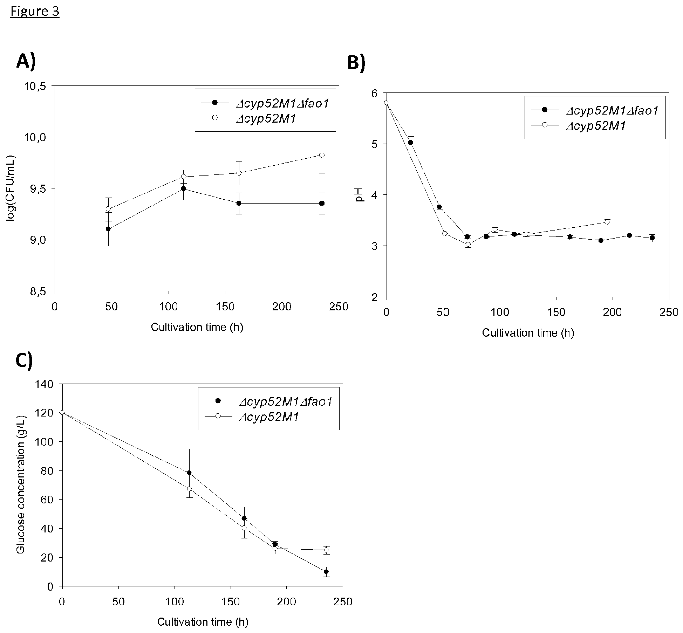

Figure 3: Important parameters for characterization of the *Δcyp52M1Δfao1* strain (●), compared to the parental *Δcyp52M1* strain (○) in shake flask. The average values and respective standard deviations are presented of A) log(CFU/mL)s, B) pH and C) glucose concentration (g/L). 1.8 (w/v) % of oleyl alcohol was added after 48 h of cultivation as hydrophobic substrate.

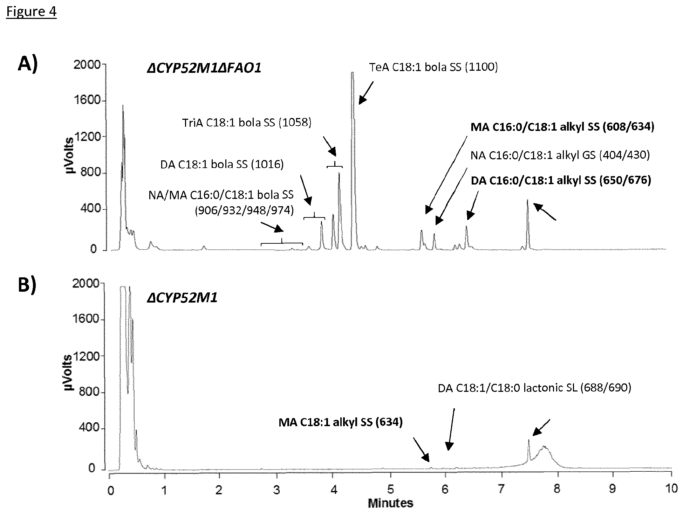

Figure 4: UPLC-ELSD chromatograms of end samples of shake flask experiments for the A) *Δcyp52M1Δfao1* strain, compared to the B) *Δcyp52M1* strain both fed with 1.8 (w/v) % oleyl alcohol (both 3x diluted). Respective masses determined with UPLC-MS are depicted above the respective peaks. In bold, masses corresponding to alkyl SS are depicted. An overview of possible glycolipid masses is given in Table 2.

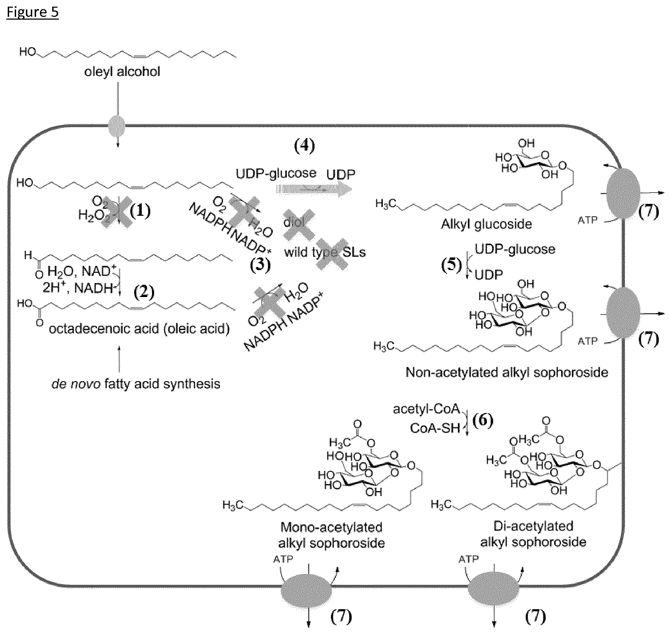

Figure 5: Proposed production pathway of alkyl sophorosides (SSs) in the *Δcyp52M1Δfao1* strain when fed with oleyl alcohol. (1) Fatty alcohol oxidase fao1, (2) fatty aldehyde dehydrogenase FAD, (3) cytochrome P450 monooxygenase CYP52M1, (4) glucosyltransferase UGTA1, (5) glucosyltransferase UGTB1, (6) acetyltransferase AT and (7) SL transporter MDR. By knocking out the *foo1* and *cyp52M1* gene (indicated by crosses), the fatty alcohol is not converted towards the corresponding fatty aldehyde, nor to the corresponding diol (in this case 1,18-octadecenediol) nor are de novo fatty acids hydroxylated (indicated by cross) to then enter the SL biosynthesis. In this way, the accumulating alcohol is directly glucosylated by glucosyltransferases UGTA1 and UGTB1, which sequentially add glucose molecules on the fatty alcohol and alkyl glucoside backbone respectively. A non-acetylated alkyl SS is obtained, which should be acetylated by the acetyltransferase AT, giving rise to a mixture of non-, mono- and di-acetylated alkyl SSs. Finally, these glycolipids are likely to be transported out of the cell, presumably by the MDR transporter responsible for secretion of wild type SLs (Van Bogaert *et al.,* 2013).

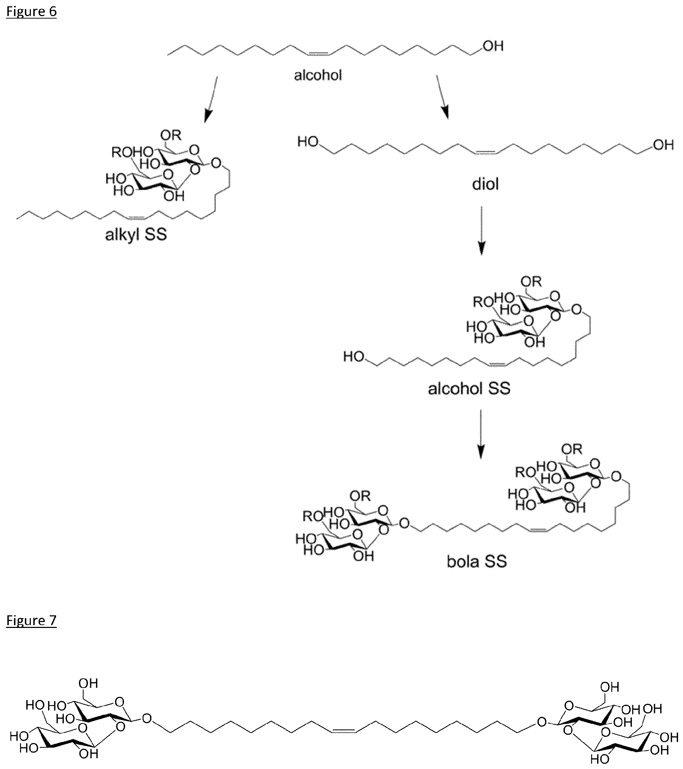

Figure 6: Overview of the production pathway using the *Δcyp52M1Δfao1* strain and feeding the latter with oleyl alcohol. By knocking out the *cyp52M1* gene, it was expected that only glycosylation could be performed as suggested in route A, giving rise to an acetylated mixture of alkyl SS (up to two acetylations), acetylations are represented as R groups. However, due to another unknown and unexpected activity of presumably a CYP52 enzyme in *S. bombicola,* hydroxylation was observed, which gave rise to acetylated bola SSs (up to four acetylations) after glycosylation. In the end, a mixture of mainly bola SSs and minor alkyl SSs was found. Hydroxylation solely occurred terminally, in contrast to the *ΔatΔsbleΔfao1* strain reported in *Van Renterghem et al.* (2018).
Figure 7: Structural confirmed composition of the purified oleyl alcohol-based (C18:1) bola SS produced by the *Δcyp52M1Δfao1* strain. Only 100 % terminal (ω) hydroxylated compounds were found.

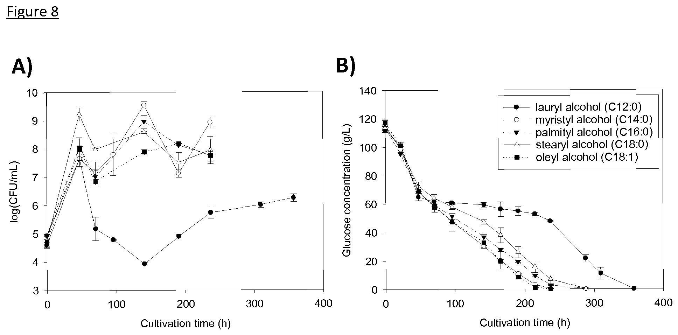

Figure 8: A) log(CFU/mL) and B) glucose concentration of the *Δcyp52M1Δfao1* strain in function of cultivation time when different primary alcohols were fed (lauryl, myristyl, palmityl, stearyl or oleyl alcohol). The average values and respective standard deviations are presented. The respective 1.8 (w/v) % of alcohol was added after 48 h of cultivation.

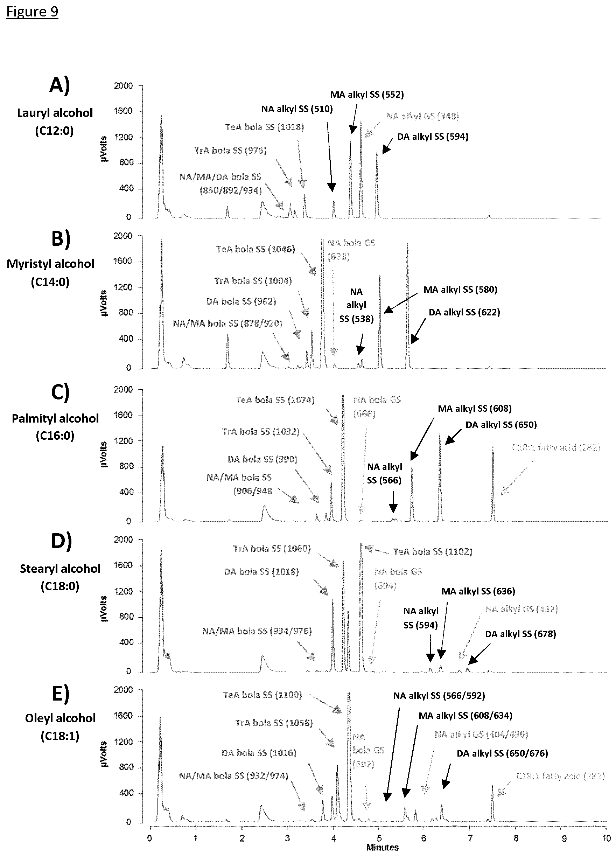

Figure 9: End UPLC-ELSD chromatograms for the *Δcyp52M1Δfao1* strain, fed with 1.8 (w/v) % (A) lauryl alcohol, (B) myristyl alcohol, (C) palmityl alcohol, (D) stearyl alcohol or (E) oleyl alcohol after 48 h of cultivation. All the end broths are 3x diluted. Retention times are indicated for the alkyl (up to two possible acetylations) and bola SSs (up to four possible acetylations). In bold, the glycolipid compounds are depicted when the respective fed alcohol was incorporated in bola SS (dark grey) and alkyl SS (black), alcohol and alkyl GS (light grey). The peaks with retention time < 1 min correspond to a mixture of strong hydrophilic compounds present in the samples as sugars, proteins and salts.

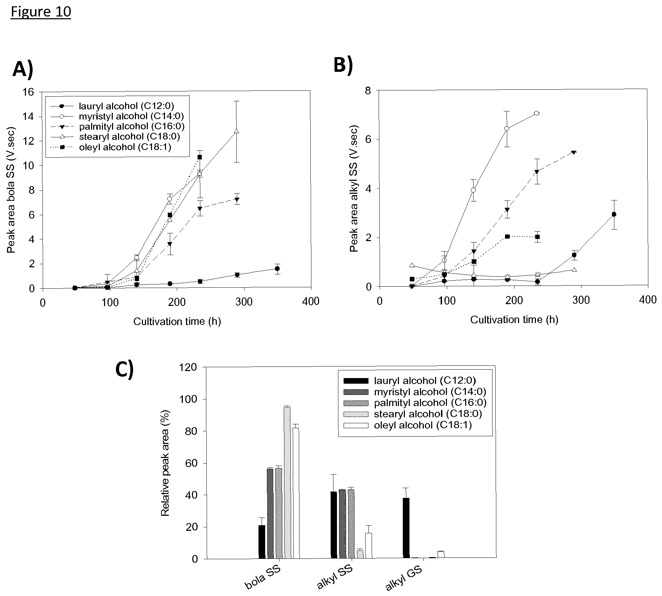

Figure 10: Bola SS (A) and alkyl SS production (B) of the *Δcyp52M1Δfao1* strain in function of cultivation time, when fed with 1.8 (w/v) % of the respective primary alcohol after 48 h of cultivation. Production is expressed as the sum of peak areas (V.sec) determined by UPLC-ELSD. (C) Relative peak areas (%) of the end broths of the *S. bombicola Δcyp52M1Δfao1* strain determined with UPLC-ELSD of the different glycolipid types, fed with 1.8 (w/v) % of lauryl alcohol (black), myristyl alcohol (dark grey), palmityl alcohol (medium grey), stearyl alcohol (light grey) or oleyl alcohol (white) after 48 h of cultivation.

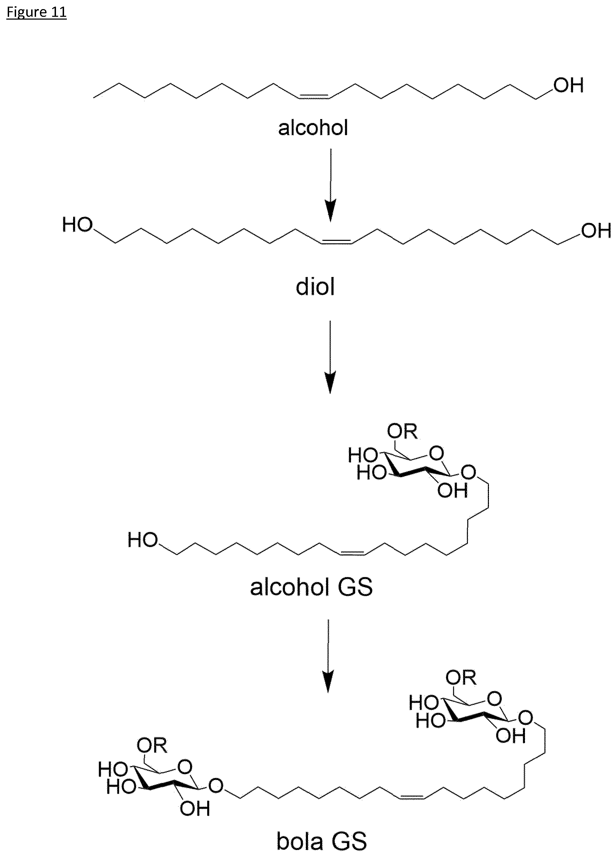

Figure 11: The triple knock out strain (ΔCYP52M1 ΔFAO1 ΔUGTB1) produces (acetylated) symmetrical bola glucosides (GS). The figure shows the pathway to synthesize(acetylated) symmetrical bola glucosides. The bottom figure shows the actual (Acetylated) symmetrical bola glucosides C18:1. R=COCH3 or H.

### Description of invention

The present invention relates to a method to produce fully symmetrical bola sophorosides which are free from contaminating sophorolipids (acidic, lactonic, bola) comprising feeding a mutated fungal strain such as a *Starmerella bombicola* strain with a fatty alcohol having an aliphatic tail chain length of at least 6 carbons wherein said fungal strain has a non-functional CYP52M1 cytochrome P450 monooxygenase and a non-functional FAO1 fatty alcohol oxidase, and wherein said fungal strain is a yeast selected from the group consisting of *Starmerella (Candida) bombicola, Candida apicola, Candida batistae, Candida floricola, Candida riodocensis, Candida stellate, Candida kuoi, Candida sp. NRRL Y-27208, Rhodotorula bogoriensis sp., Wickerhamiella domericqiae* and a sophorolipid-producing strain of the *Starmerella* clade.

The term 'a fully symmetrical bola sophoroside' refers to a bola sophoroside (i.e. a sophoroside consisting of two sophorose units located on each side of the fatty hydrophobic linker or aliphatic tail as described by Van Renterghem et al. (2018)) wherein both sophorose entities or units are attached in an exclusively terminal position (ω position) on the fatty hydrophobic linker.

The fatty (hydrophobic) linker or aliphatic chain can contain at least 6 (i.e. 6, 7, 8, 9, 10, 11, 12, 13, 14, 15, 16, 17, 18, 19, 20 or more) carbon atoms, can be branched and/or can contain insaturations.

The terms 'free from contaminating sophorolipids' refers to the production of fully symmetrical bola sophorosides wherein no measurable amounts of acidic- bola- and/or lactonic sophorolipids are produced as contaminating by-products.

The term 'fungal strain' refers to any genetic variant or sub-type of a particular fungus, such as a yeast strain, which is capable of producing sophorolipids. In other words, the present invention relates to a usage as described above wherein said fungal strain is a fungal strain capable of producing sophorolipids endogenously or after genetic modification. This fungal strain is a fungal species selected from the group consisting of: *Candida apicola* (Gorin *et al.,* 1961) which was initially identified as *C*. *magnolia, C. bombicola* (Spencer *et al.,* 1970), *Wickerhamiella domericqiae* (Chen *et al.,* 2006), *Rhodotorula bogoriensis* (Tulloch *et al.,* 1968), *Candida batistae* (Konishi *et al.,* 2008), *Candida floricola* (Imura *et al.,* 2010), *Candida riodocensis, Candida stellate* and *Candida sp.* NRRL Y-27208 (Kurzmanet *al.,* 2010), *Candida kuoi* (Kurtzman, 2012) and any other strain of the *Starmerella* clade. In embodiments, said *Starmerella* (*Candida) bombicola* is the strain *Starmerella* (*Candida) bombicola* ATCC 22214 (CBS 6009) or strains derived thereof.

The terms "a mutated fungal strain" relates to a fungal strain as defined above wherein said strain is mutated so that the enzymes CYP52M1 and FAO1 are non-functional. With regard to CYP52M1 this means that no hydroxylation of the fatty alcohol substrate can occur anymore and with regard to FAO1 this means that no oxidation of the OH group present on the fatty alcohol towards the corresponding aldehyde can occur anymore.

The term 'dysfunctional' means in general a gene or protein which is not performing 'normally', and/or, has an absent or impaired function. The term thus refers to a gene or protein which is: a) not functional because it is not present, b) still present but is rendered non-functional or c) which is present but has a weakened or reduced function. The term 'dysfunctional' specifically refers to a gene having lost its capability to encode for the fully functional enzymes CYP52M1 and FAO1, or polypeptides/proteins having lost its CYP52M1 and FAO1 activity, either completely or partially. 'Partially' means that the activity of the latter enzymes -measured by any method known in the art- is significantly lower (p < 0.05) when compared to the activity of the wild-type counterparts of said enzymes.

A 'dysfunctional' nucleic acid molecule as defined above can be obtained by mutation or by any known means to silence the transcription or translation of said nucleic acid. The latter means comprise the insertion of a nucleic acid fragment, a marker gene or any other molecule in the functional coding or non-coding part of the target gene, a mutation or removal of the functional coding or non-coding part of the target gene, the usage of specific siRNAs, miRNAs or combinations thereof, or any other means known to a skilled person.

The term 'mutation' refers to a spontaneous mutation and/or to an induced mutation in the genome of said fungal strain. Said mutation can be a point mutation, deletion, insertion or any other type of mutation.

Similarly, a 'dysfunctional' polypeptide as defined above can be obtained by any (small) compound or other means to weaken or disrupt the function of the target genes of the present invention. Means to silence the transcription or translation or means to disrupt the function of the target genes of the present invention or means to disrupt the function of a necessary regulator/activator protein of the target genes comprise the usage of any molecule such as -but not limited to- an antibody, an amino acid, a peptide, a small molecule, an aptamer, a ribozyme, an oligoribonucleotide sequence such a dsRNA used to initiate RNA interference (RNAi) or an anti-sense nucleic acid. Such a molecule is thus capable to bind on a target protein or an activator/regulator protein thereof, or, is capable to interfere with the cellular synthesis of the target enzyme or of an activator/regulator thereof by -for example-binding and degrading mRNA's encoding for a target protein or an activator/regulator thereof.

A 'dysfunctional' CYP52M1 and FAO1 refers to an enzyme with reduced activity, obtained by any method known by the person skilled in the art. Non-limiting examples of said methods are the introduction of point mutations, the usage of truncated or mutated enzymes, the usage of inhibitors or antibodies, and any of the methods described above.

The term 'dysfunctional' thus also refers to the absence of the specific genes mentioned above *(cyp52M1* and *fao1*) in the genome of the applied fungal strain.

The genes and their encoded enzymes CYP52M1 and FAO1 are well known in the art and are -for example- described in WO2011154523 (CYP52M1) and in Takahashi et al. (2016) (FAO1).

In embodiments, the present invention relates to a method as described above wherein the gene encoding for the CYP52M1 cytochrome P450 monooxygenase and the gene encoding for the FAO1 fatty alcohol oxidase are knocked out.

The present invention further relates to a method as described above wherein said alcohol having an aliphatic tail chain length of at least 6 carbons is hexanol, octanol, decanol, undecyl alcohol, lauryl alcohol, tridecyl alcohol, myristyl alcohol, pentadecyl alcohol, cetyl alcohol, palmitoleyl alcohol, heptadecyl alcohol, stearyl alcohol, isostearyl alcohol, oleyl alcohol, nonadecyl alcohol, arachidyl alcohol or a mixture thereof.

The present invention also relates to a method as described above, wherein said fully symmetrical bola sophorosides are (tetra-, tri-, di- or mono-) acetylated.

With the term 'acetylated' is specifically meant bola- or alkyl sophorosides which contain an 'acetyl' functionality on position 6' or 6" of one or both of the two sophorose moieties present in said bola or alkyl sophorosides. The term 'acetylation' (or ethanoylation) more generally describes a reaction that introduces an acetyl functional group into a chemical compound resulting in an acetoxy group i.e. the substitution of an acetyl group for an active hydrogen atom. A reaction involving the replacement of the hydrogen atom of a hydroxyl group with an acetyl group (CH₃ CO) yields a specific ester, the acetate.

The present invention also relates to a mutated fungal strain such as a *Starmerella bombicola* strain having a non-functional CYP52M1 cytochrome P450 monooxygenase and a non-functional FAO1 fatty alcohol oxidase, and wherein said fungal strain is a yeast selected from the group consisting of *Starmerella (Candida) bombicola, Candida apicola, Candida batistae, Candida floricola, Candida riodocensis, Candida stellate, Candida kuoi, Candida sp. NRRL Y-27208, Rhodotorula bogoriensis sp., Wickerhamiella domericqiae* and a sophorolipid-producing strain of the *Starmerella* clade.

This latter strain can be made by any method known in the art and as is described above. A specific but non-limiting method to create a CYP52M1 cytochrome P450 monooxygenase and FAO1 knockout strain is described further in the 'materials and methods' section of the 'Examples' section.

In embodiments, the gene encoding for the CYP52M1 cytochrome P450 monooxygenase and the gene encoding for the FAO1 fatty alcohol oxidase are knocked out.

The present invention further relates to the usage of a mutated fungal strain such as a *Starmerella bombicola* strain as described above to produce fully symmetrical bola sophorosides which are free from contaminating fatty acid containing sophorolipids as is described above.

The present invention also relates to the usage of a mutated fungal stain such as a *Starmerella bombicola* strain as described above to produce fully symmetrical diols. The latter diols are the precursors of the symmetrical bola sophorosides as is shown in Fig 6.

The present invention further relates to the usage of a mutated fungal strain such as a *Starmerella bombicola* strain as described above wherein said symmetrical bola sophorosides are part of a mixture further comprising alkyl sophorosides.

The latter mixtures are formed because some fatty alcohol molecules are glycosylated by subsequent action of the enzymes UGTA1 and UGTB1 (see also further) and then secreted before the fed fatty alcohol could be hydroxylated. This gives rise to alkyl sophoroside production as 'unfinished' bola sophorosides or other bola sophorosides intermediates together with the production of bola sophorosides.

Hence, the present invention also relates to the usage of a mutated fungal strain such as a *Starmerella bombicola* strain as described above to produce alkyl sophorosides.

Indeed, the latter alkyl sophorosides are produced when the fed fatty alcohol is not hydroxylated at the other side of the aliphatic chain and glycosylated and secreted as alkyl sophorosides.

The present invention further relates to a mutated fungal strain as described above, wherein said fungal strain further comprises a non-functional glucosyltransferase (UGTB1) that is responsible for the second glucosylation step in the sophorolipid/sophoroside biosynthetic pathway.

The term 'A glucosyltransferase that is responsible for the second glucosylation step in the sophorolipid biosynthetic pathway' is described in detail in WO2011154523. Indeed WO2011154523 discloses that there is a first glycosylation (see example 2 of WO2011154523) and a second glycosylation step (see example 3 of WO2011154523) in the sophorolipid pathway wherein a 'first' (i.e. UGTA1) and a 'second' glycosyltransferase (i.e. UGTB1 having Genbank Accession number HM440974 and as is also described in detail in Saerens et al. 2011 (Yeast: 279-292)), are involved.

The term 'dysfunctional' is described above.

The present invention also relates to the use of mutated fungal strain as described above to produce symmetrical bola glucosides.

The term 'symmetrical bola glucosides' relates to a glucoside with one glucose molecule at each side of the fatty hydrophobic linker or aliphatic tail (or chain) through two terminal (ω) glycosidic linkages as described above.

The present invention relates to both 'acetylated' and 'non-acetylated' symmetrical bola glucosides but specifically relates to the use of mutated fungal strain as described above wherein said symmetrical bola glucosides are acetylated.

The term 'acetylated' is described above.

### Examples

### Materials and Methods

### Strains, media and culture conditions

As parental strain, the SL deficient *Δcyp52M1* strain *S. bombicola* was used (Van Bogaert *et al.,* 2013a). A *S. bombicola* fatty alcohol oxidase *fao1* knockout was obtained similarly as described by Van Renterghem (2018) by integrating the *ura3* gene under the regulatory control of its own promotor and tyrosine kinase (*tk*) terminator at the respective *fao1* loci in the *Δcyp52M1* strain. Three transformant colonies of the new strain were evaluated in terms of growth and glycolipid production, in parallel with the *Δcyp52M1* parental strain. Cultivation, selection and transformation were performed as described by Lodens *et al.* (2018).

Production experiments using *S. bombicola* were performed using the production medium described by Lang *et al.* (2000). For shake flask experiments, 5 mL tube cultures were set up 24 h (30°C), before transferring to shake flasks (4 % inoculation). Production experiments were executed with feeding of fatty alcohols: oleyl alcohol, stearyl alcohol, lauryl alcohol, myristyl alcohol or cetyl alcohol which were added after 48 h of cultivation. The *Δcyp52M1Δfao1* strain was also assessed without addition of the hydrophobic alcohol. For every production experiment, cultivation was stopped when glucose was depleted from the medium. Experiments were performed in duplicate, and average values with standard deviations are presented.

### Molecular techniques

### General techniques

General molecular techniques were employed as described by Green and Sambrook (2012). Linear deletion cassettes were generated from vector backbones cloned and maintained in *E. coli,* based on the pGEM-T (Promega) and pJET (Thermo Fisher) vectors and cloning steps are described below. All primer sequences are represented in Table 1 and a graphical representation of the strain construction is shown in Figure 2.

**Table 1: Primers used for the creation of knockout strain of the S. bombicola fatty alcohol oxidase gene (fao1) in the Δcyp52M1 strain.**

| **Primer names** | **'5-sequence-3'** |
|---|---|
| P30_FOR_checkpromlN | AAGGCGGGCTGGAATGCATATCTGAG (SEQ ID NO 1) |
| P34_REV_checkcasslN | GATGTCGAATAGCCGGGCTGCTAC (SEQ ID NO 2) |
| P276_pJET_Rev | AAGAACATCGATTTTCCATGGCAG (SEQ ID NO 3) |
| P625_pJET_For | CGACTCACTATAGGGAGAGCGGC (SEQ ID NO 4) |
| P1001_FOR_FAO1_extgibpJET | CTCGAGTTTTTCAGCAAGATTGCCAAGTCGTTCAACACAG (SEQ ID NO 5) |
| P1002_REV_FAO1_extgibpJET | AGGAGATCTTCTAGAAAGATCTGAGACAGCAGCTTGTCAC (SEQ ID NO 6) |
| P1003_FOR_pJETextGib_FAO1 | GTGACAAGCTGCTGTCTCAGATCTTTCTAGAAGATCTCCTAC (SEQ ID NO 7) |
| P1004_REV_pJETextGib_FAO1 | CTGTGTTGAACGACTTGGCAATCTTGCTGAAAAACTCGAGCCA TC (SEQ ID NO 8) |
| P1020_FOR_FAO1 | TGCCAAGTCGTTCAACACAG (SEQ ID NO 9) |
| P1021_REV_FAO1 | CTGAGACAGCAGCTTGTCAC (SEQ ID NO 10) |
| P1022_REV_checkFAO1KO | GCCTTGGCATTCAACATCTCAGGGAATC (SEQ ID NO 11) |
| P1023_FOR_checkFAO1KO | GCACGCCCTTAGCTTCAGAG (SEQ ID NO 12) |
| P1062_FOR_Pura3_extgib_upFAO1 | GACTGAGATGACGGAAGAGGCCCGAACATACCAGTTTCGC (SEQ ID NO 13) |
| P1063_REV_tTK_extgib_downFAO1 | AAGCTTAGTGAGATCCGCGTGAACAAACGACCCAACACCC (SEQ ID NO 14) |
| P1064_FOR_downFAO1_extgibtTK | GGGTGTTGGGTCGTTTGTTCACGCGGATCTCACTAAGCTTC (SEQ ID NO 15) |
| P1065_REV_upFAO1_extgibPura3 | GCGAAACTGGTATGTTCGGGCCTCTTCCGTCATCTCAGTC (SEQ ID NO 16) |
| P1347_FOR_FAO1_up_check | GCCAGTGCAACAAGTATGAG (SEQ ID NO 17) |
| P1348_REV_FAO1_down_check | GACCAGGCTAAACGCATCAC (SEQ ID NO 18) |

### Creation of the Δcyp52M1Δfao1 knockout strain

The creation of the *fao1* knockout cassette is described Van Renterghem *et al.* (2018) and was used to transform the *S. bombicola Δura3::0Δcyp52M1::Pgapd_hph_Ttk* strain, or further on called the Δ*ura3Δcyp52M1* strain. The *hph* gene was isolated from *Streptomyces hygroscopus,* and encodes for hygromycin B phosphotransferase resistance (Gritz & Davies, 1983). After transformation, the *ura3* positive colonies were selected on selective SD medium. Correct integration of the cassette was confirmed by colony PCR. For the newly-created *Δura3::O Δcyp52M1::Pgapd_hph_tTK Δfao1::Pura3_ura3_Ttk* strain, further on called *Δcyp52M1Δfao1,* respectively, three successful colonies were chosen.

### Downstream processing and characterization

The purification of the generated products of the *Δcyp52M1Δfao1* strain when fed with oleyl was done by performing alkaline hydrolysis (pH 12, 5 M NaOH, 37°C, 1 h) to fully deacetylate the glycolipids to obtain a more uniform product. The purified and dried product was further fractionated by preparative liquid chromatography (PLC) for NMR analysis (see below).

### Preparative layer chromatography (PLC)

Uniplate 20x20 cm PLC plates coated with 2 mm silica gel, impregnated with a green fluorescent indicator (F254) (Analtech) were used. First, 100 mg of sample was dissolved in MilliQ water. Subsequently, the solution was applied as a long streak at 2 cm from the bottom of the plate. The PLC plate was put in a solvent chamber containing the SL solvent mixture chloroform/methanol/water (65/15/2, v/v/v) (Asmer *et al*., 1988). After solvent development and respective evaporation, the plate was put under UV light at 254 nm. Subsequently, the highlighted zone of interest was scraped off using a scalpel and the scraped-off silica gel was collected. The compound was subsequently resolved by adding 20 mL MilliQ water to the falcon and centrifuged for 10 minutes at 4500 rpm. The supernatant was collected, and the process was repeated. The total supernatant, containing the bola SS product, was filtered (cut-off 0.22 µm, Millex^{®} GV) to remove residual silica gel particles. Finally, the water was removed by using an Alpha 1-4 lyophilisator (Christ) to obtain a dry and highly-pure product, suitable for NMR analysis.

### Analytical techniques

### Follow-up of growth

Optical density (OD) of cultures was measured at 600 nm using the Jasco V 630 Bio spectrophotometer (Jasco Europe) of 1 mL samples diluted with physiological solution (9 g/L NaCl). The viability of yeast cells in cultivation experiments was assessed by determining colony forming units (CFUs) which were expressed as the average logarithm of CFUs per culture volume as log(CFU/mL) (Saerens *et al.,* 2011). Alternatively, Cell Dry Weight (CDW) was determined by centrifuging 1 mL fermentation samples at 14000 rpm for 5 min in tared Eppendorf tubes, and subsequently washing the cell pellets twice with 1 mL physiological solution. The remaining cell pellet was put into a 70°C oven for 5 days and then weighed afterwards. The CDW (g/L) was calculated after subtraction of the empty weight of the Eppendorf tube.

### Follow-up of glucose concentration

Glucose concentrations were determined using the 2700 Select Biochemistry Analyser (YSI Inc.) or using Ultra Performance Liquid Chromatography (Waters Acquity H-Class UPLC), coupled with an Evaporative Light Scattering Detector (Waters Acquity ELSD Detector) (UPLC-ELSD). For the UPLC analysis, an Acquity UPLC BEH Amide column (130 Å, 1.7 µm, 2.1 × 100 mm) (Waters) was used at 35°C and an isocratic flow rate of 0.5 mL/min of 75 % acetonitrile and 0.2 % triethylamine (TEA) was applied (5 min/sample). For the ELS detection, the nebulizer was cooled to 15°C and the drift tube was kept at a temperature of 50°C. The linear range was found to lie between 0 and 5 g/L glucose, using a gain of 100 for ELS detection (Empower software). To express glucose consumption, a linear curve was fitted through the obtained glucose concentrations by UPLC-ELSD, and the respective slope was taken and denoted as the glucose consumption rate (g/L.h).

### Analysis of glycolipids

Samples for glycolipid analysis were prepared by shaking a mixture of 1 mL of pure ethanol and 0.5 mL of fermentation broth vigorously for 5 minutes. Subsequently, after centrifugation for 5 minutes at 15000 rpm, the cell pellet was removed and the supernatant was filtered using a PTFE filter (cut-off 0.22 µm, Novolab) and adequately diluted in 50 % ethanol (unless stated otherwise) before analyzing on (Ultra) High Pressure Liquid Chromatography - Mass Spectrometry ((U)HPLC-MS) and (U)HPLC-ELSD (Evaporative Light Scattering Detector).

HPLC-MS was performed using an LC (Shimadzu), coupled to an MS (Micromass Quattro LC) detection system. The different components were separated by polarity on a Chromolith Performance RP-18 Endcapped 100-4.6 mm column (Merck KGaA) at 30°C. The LC-MS method uses a gradient elution based on two solvents: MilliQ with 0.5 % acetic acid, and pure acetonitrile (ACN). During the analysis, a flow rate of 1 mL/min was applied. The gradient starts with 5 % acetonitrile and increases linearly until 95 % over the course of 40 min. After this, the 95 % acetonitrile is held for another 10 min, after which this is brought back to 5 % acetonitrile in 5 min. The total analysis time per sample is 60 min/sample. The scanning range of the MS was set to 215-1100 g/mol. Using similar conditions as mentioned for HPLC-MS, HPLC-ELSD analysis was performed by Varian Prostar HPLC (ThermoScientific), coupled with an 2000ES ELSD (Alltech) at 40°C. All other conditions are similar as mentioned for the HPLC-MS.

UPLC-ELSD analysis was performed on a Acquity H-Class UPLC (Waters) and Acquity ELSD Detector (Waters), employing the same column and analysis method as UPLC-MS. For the ELSD detection, the nebulizer was cooled at 12°C and the drift tube was kept at a temperature of 50°C, the gain was set to 200. To quantify the glycolipids, a dilution series of purified product was prepared. An available C18:1 acetylated bola SS purified batch (batch number SL24A) and purified acetylated C16:0 alkyl SS batch (batch number aAlkC16_2) were employed for quantification of bola and alkyl SS, respectively.

Alternatively, UPLC-MS was performed with an Accela (ThermoFisher Scientific) and Exactive Plus Orbitrap Mass Spectrometer (ThermoFisher Scientific). For glycolipid analysis, an Acquity UPLC CSH C18 column (130 Å, 1.7 µm, 2.1 mm × 50 mm) (Waters) and a gradient elution system based on 0.5 % acetic acid in milliQ (A) and 100 % acetonitrile (B) at a flow rate of 0.6 mL/min was applied. The method was as follows: the initial concentration of 5 % B (95 % A) increases linearly until 95 % B (5 % A) during the first 6.8 min, and then linearly decreases again to 5 % B (95 % A) during 1.8 min. Subsequently, 5 % B (95 % A) is maintained until the end of the run (10 min/sample). Negative ion mode was used, and 2 µL samples were injected. MS detection occurred with a Heated Electrospray Ionization (HESI) source and conditions were set to detect masses ranging from 215-1300 m/z in a qualitative way.

All ¹H and ¹³C NMR spectra were recorded at 400 and 100.6 MHz, respectively, on a Bruker Avance III, equipped with ¹H/BB z-gradient probe (BBO, 5 mm). DMSO-[D6] was used as solvent, and as internal chemical shift standard (2.50 ppm for ¹H and 39.52 ppm for ¹³C). All spectra were processed using TOPSPIN 3.2 software. Attached Proton Test (APT), ¹³C, COSY, and HSQC spectra were acquired through the standard sequences available in the Bruker pulse program library. Custom settings were used for HMBC (32 scans), TOCSY (100 millisec MLEV spinlock, 0.1 sec mixing time, 1.27 sec relaxation delay, 16 scans), and H2BC (21.8 millisec mixing time, 1.5 sec relaxation delay, 16 scans), according to literature (Gheysen, Mihai, Conrath, & Martins, 2008; Petersen et al., 2006).

### Statistical analysis

When two different groups were compared, the Welch's test was performed with a 95 % confidence level using GraphPad Prism 7.04 software. For multiple group comparisons, analysis of variance (ANOVA) with Bonferroni's multiple comparison test correction with a 95 % confidence level using GraphPad Prism 7.04 software was employed. For parameters pH, CFU, OD and glucose consumption, average values were taken when the yeast cells attained their stationary phase (after 48 h of cultivation). In general, data represented in graphs are the average and standard deviation of two experimental replicates (unless stated otherwise).

### Results

### Construction of knockout strains

The fatty alcohol oxidase *fao1* knockout cassette (described in Van Renterghem *et al.,* 2018) was used to transform the *S*. *bombicola Δcyp52M1Δura3* strain. After selection of the *ura3⁺* colonies on selective SD plates, correct integration at both sides of the knockout cassette was controlled by performing colony PCR using two primer combinations (see Table 1). Three correct colonies of the newly created *Δcyp52M1Δfao1* strain were selected for further characterization. The three selected transformants of the novel strain behaved similar to each other in terms of OD, CFUs, glucose consumption and glycolipid production. Therefore, only one colony is discussed in the next section for comparison with the parental *Δcyp52M1* strain in terms of growth, pH, glucose consumption and glycolipid production.

### Initial characterization of knockout strain

For the wild type *S. bombicola,* colza oil (60-80 % oleic acid) or pure oleic acid results in the best SL titers (Asmer *et al.,* 1988; Rau *et al.,* 2001) i.e. a hydrophobic substrate with C18 carbon chain length. Therefore, the newly created *fao1* knockout strain was first assessed on shake flasks fed with a C18 fatty alcohol, i.e. oleyl alcohol (C18:1) as hydrophobic substrate.

### Growth, pH and glucose consumption

Important parameters such as log(CFU/mL), pH and glucose consumption are depicted in Figure 3. Average values and standard deviations of assessed duplicates are presented. OD₆₀₀ evolution was not presented, due to interferences of the measurements with the oleyl alcohol substrate. In terms of log(CFU/mL) values in function of cultivation time (Figure 3A), no significantly different CFU values were obtained between the new strain and reference strain. The observed pH drop was equal (Figure 3B). The glucose consumption rate for the *Δcyp52M1Δfao1* and parental strain are similar (0.47 ± 0.06 and 0.47 ± 0.01 g/L.h), as presented in Figure 3C.

### Glycolipid production

When looking at glycolipid production of the assessed strain, depicted in Figure 4, distinctly different production profiles for the *Δcyp52M1Δfao1* strain can be observed compared to the *Δcyp52M1* parental strain.

The parental *Δcyp52M1* strain (Figure 4B) produced none (or very minor) amounts of glycolipids (< 7 min). A small peak corresponding to oleic acid (282 g/mol) is visible at later retention times (> 7 min) (similarly as the one observed for the *Δcyp52M1Δfao1* strain). The fact that almost no alkyl SS are detected, indicates that the fed oleyl alcohol is mainly oxidized to the corresponding oleic acid by the still functional ω-oxidation pathway (as *FAO* is not knocked out). Subsequently, as hydroxylation of oleic acid is inhibited by the *cyp52M1* knockout, the glucosyltransferases cannot glycosylate the fatty acid, so the oleic acid accumulates or is broken down by the β-oxidation.

In contrast to the parental strain, for the *Δcyp52M1Δfao1* strain (Figure 4A) glycolipid production is clearly observed. Significant amounts of glycolipids were found at retention times between 3.0 - 4.5 min and minor amounts between 5.5 - 6.5 min. After UPLC-MS analysis, surprisingly it was found that the masses in the early retention range (3-4.5 min) actually corresponded to bola SSs (900-1100 g/mol range), instead of the aimed for alkyl SS (550-700 g/mol range). Alkyl SS were indeed detected, but only in the abovementioned minor amounts between 5.5 - 6.5 min. This synthesis of bola SSs is theoretically (based on the art) impossible in this strain, as diol formation (see Figure 5) (necessary for bola SS synthesis) through hydroxylation of the fed fatty alcohol should not be possible due to the deletion of the *cyp52M1* gene. Indeed, no such action is observed in the parental strain (see Figure 4B), which as mentioned in the art indeed does not show any oxidation/hydroxylation activity towards oleyl alcohol (see Figure 4B) nor on oleic acid and/or rapeseed oil (Van Bogaert *et al.,* 2013).

Thus, surprisingly, the *Δcyp52M1Δfao1* strain mainly produces bola SSs when fed with oleyl alcohol (Figure 6), corresponding to the early retention times (3.0 - 4.5 min). As the AT gene is not knocked out in this strain (in contrast to the strain designed to produce bola SSs (Soetaert *et al.,* 2013; Van Renterghem *et al.,* 2018), acetylation of the bola SSs is also observed for this new strain described above. The tetra-acetylated C18:1 bola SS (1100 g/mol) at 4.4 min is the most abundant component of the bola SSs, followed by the less abundant tri- and di-acetylated ones (1016 and 1058 g/mol, respectively). Non- and mono-acetylated C18:1 bola SS were also detected (respective 932 and 974 g/mol). The fact that the mono-, di- and tri-acetylated bola SS appear in two different peaks at 4.05 and 4.15 min, is probably explained by a different acetylation pattern of the molecule, similarly as shown for acidic SLs shown in literature (Davila *et al.,* 1993; Saerens *et al.,* 2011; Saerens, 2012). Besides C18:1 bola SSs, also C16:0 based SSs (906 g/mol) are detected arising from the C16 amounts in the fed oleyl alcohol substrate (contaminating amounts of 2 - 10 % cetyl alcohol or 1-hexadecanol are present in the substrate). Minor amounts of alkyl SSs were also produced and mainly C18:1 non-, mono- and di-acetylated alkyl SSs were detected (592/634/676 g/mol, respectively), but also traces of C16:0 alkyl SSs were found (566/608/650 g/mol, respectively).

It thus appears that the fed alcohol is favorably hydroxylated to the corresponding diol, and as such goes through the glycosylation cycle of UGTA1 and UGTB1 twice, to give rise to bola SSs (see Figure 6), instead of just accumulating into alkyl SSs. As the parental strain fed with alcohols did not produce these compounds when fed on oleyl alcohol, it appears that the combination of the *cyp52M1* and*fao1* knockout is crucial.

To enable detailed characterization of the produced compounds, the bola SSs and alkyl SSs were purified from the mixture as described.

### NMR structure analysis

NMR analysis was performed on a purified non-acetylated C18:1 bola SSs standard derived from the broth of the *Δcyp52M1Δfao1* strain.

Surprisingly, NMR analysis showed that in the produced bola SSs, both sophorose moieties exclusively linked in a terminal (ω) fashion to the hydrophobic linker, presented in Figure 7. A full symmetrical glycolipid molecule is thus obtained, with chemical formula C₄₂H₇₆O₂₂.

### Feeding different chain lengths of fatty alcohols

Unexpectedly, no successful uniform production of either bola SSs or alkyl SSs was obtained for the *Δcyp52M1Δfao1* strain when feeding with oleyl alcohol. Instead, mixtures of both were found, although the majority of the novel glycolipids were (acetylated) bola SSs and only minor amounts of alkyl SS were produced. The production of tetra-acetylated bola SS was unexpected, since the CYP52M1 enzyme was thought to be the only enzyme involved in glycolipid production thus far in S. *bombicola.* To assess the influence on the chain lengths of primary alcohols to the glycolipid production, different substrates were fed to the *Δcyp52M1Δfao1* strain. The parental strain *Δcyp52M1* was assessed in parallel. Medium and long-chain alcohols were selected between a chain length of 12 and 18, similarly as reported by *Davila et al.* (1994) for alkanes. Similarly as for the experiments described above, the hydrophobic substrate was added after 48 h of cultivation.

### Growth, pH and glucose consumption

The addition of lauryl alcohol, myristyl alcohol, cetyl alcohol or oleyl alcohol did not significantly influence the CFU values for the *Δcyp52M1* strain compared to the *Δcyp52M1Δfao1* strain (Figure 8). However, a significant drop in CFUs was noticeable when lauryl alcohol was added, in contrast to all other fed substrates (Figure 8A). Significant higher glucose consumption rates were found when myristyl or oleyl alcohol were fed compared to cetyl or stearyl alcohol (0.48 g/L.h vs 0.38 g/L.h). After addition of lauryl alcohol (48 h after inoculation), glucose consumption stopped. Only for the *Δcyp52M1Δfao1* strain, the glucose consumption initiated again (0.33 g/L.h) 100 h after substrate addition, until all glucose was depleted. This is in line with the CFUs evolution (Figure 8A).

The *Δcyp52M1Δfao1* strain was also studied when no hydrophobic substrate was added. The CFUs and glucose consumption were not significantly different from the cultures fed with alcohols (except lauryl alcohol).

### Glycolipid production

Before addition of the primary alcohol, none of the *Δcyp52M1Δfao1* and *Δcyp52M1* cultures showed quantifiable glycolipid production. This was expected, as *de novo* fatty acids cannot be implemented into the glycolipid production pathway due to the *cyp52M1* knockout, and the alcohol has to be fed in order to initiate novel glycolipid production.

After addition of the hydrophobic substrate for the *Δcyp52M1* strain no peaks were visible after analysis on ELSD, which is in line with the expectations and with wat has been described before (Van Bogaert, 2010). Glycolipid end production profiles of the *Δcyp52M1Δfao1* strain for the different fed primary alcohols analyzed on UPLC-ELSD are represented in Figure 9. For each alcohol that was fed, a mixture of acetylated bola and alkyl SSs was produced. As expected, all produced glycolipids were originating from the fed alcohol directly. The latter is due to the fact that the *cyp52M1* gene is knocked out, hence eliminating implementation of any *de novo* (mainly C16-C18) or generated (possible oxidation of the alcohol) fatty acids.

For lauryl alcohol, from non- up to tetra-acetylated C12:0 bola SS could be detected (580/934/976/1018 g/mol). Besides C12:0 bola SS, also non- to di-acetylated C12:0 alkyl SS (510/552/594 g/mol) were found at later retention times. However, the most abundant peak in the chromatogram corresponded to C12:0 alkyl glucosides (GS) (348 g/mol) which situated in between the mono- and di-acetylated alkyl SS peaks.

For myristyl and cetyl alcohol, very similar production profiles were observed. Clear retention times corresponding to C14:0 or C16:0 bola SSs (3.00 - 4.00 min and 3.00 - 4.3 min) and C14:0 or C16:0 alkyl SSs (4.5 - 5.8 min and 5.2 - 6.5 min) could be distinguished with respective acetylations. C14:0 and C16:0 non-acetylated alkyl GS (376 and 404 g/mol) were detected using MS,. Minor quantities of di-acetylated C14:0/or C16:0 alcohol SS (or bola GL) (638 or 666 g/mol) were detectable.

When the *Δcyp52M1Δfao1* strain is fed with stearyl alcohol, again mixed production of acetylated C18:0 bola SSs (934/976/1018/1060/1102 g/mol) and C18:0 alkyl SS (594/636/678 g/mol) is observed. Additionally, minor amounts of C18:0 alkyl GS (432 g/mol) were detected. Similarly as for myristyl and cetyl alcohol, di-acetylated C18:0 alcohol SSs (or bola GL) (694 g/mol) were detected.

Addition of oleyl alcohol resulted in the glycolipid production profile described above. Also non-, mono- and di-acetylated C16:0 and C18:1 alkyl SS (566/608/650 g/mol and 592/634/676 g/mol respectively) and non-acetylated C16:0 and C18:1 alkyl GS (404 and 430 g/mol) were detected. Similarly as for the other alcohols, di-acetylated C18:1 alcohol SS (or bola GL) (692 g/mol) was also detected.

Generating the triple knock out strain (ΔCYP52M1 ΔFAO1 ΔUGTB1) for the production of symmetrical bola glucosides.

For the generation of the triple knock out strain (ΔCYP52M1 ΔFAO1 ΔUGTB1), the *ura3* marker was first removed from the double knock out strain (ΔCYP52M1 ΔFAO1) as described by Lodens *et al.,* 2019 ( Biotechnology and Bioengineering: 1-13). A linear UGTB1 knock-out cassette with *ura3* marker was generated from plasmid "pGKO ugtB1" described by Saerens et al, 2011 using PfuUltra High Fidelity PCR (Stratagene) and the primerpair GTII-472F and GTII+239R (GTII -472For : 5' - GAGAGTGGGACCTGATTC-3' (SEQ ID N° 19)/ GTII +239Rev: 5' -CTGCTCTCAACACCGAGTGTAG-3' (SEQ ID N° 20)). This deletion cassette was transformed into the *ura3* negative ΔCYP52M1 ΔFAO1 strain and correct transformants were selected. The resulting strain produces (acetylated) symmetrical bola glucosides as shown in Figure 11.

### References

Asmer, H.-J. J., Lang, S. S., Wagner, F., & Wray, V. (1988). Microbial production, structure elucidation and bioconversion of sophorose lipids. American Oil Chemists' Society, 65(9), 1460-1466.
Davila, A.-M. M., Marchal, R., & Vandecasteele, J.-P. P. (1994). Sophorose lipid production from lipidic precursors: Predictive evaluation of industrial substrates. Journal of Industrial Microbiology, 13(4), 249-257. https://doi.org/10.1007/BF01569757
Davila, A. M., Marchal, R., Monin, N., & Vandecasteele, J. P. (1993). Identification and determination of individual sophorolipids in fermentation products by gradient elution high-performance liquid chromatography with evaporative light-scattering detection. Journal of Chromatography A, 648(1), 139-149. https://doi.org/10.1016/0021-9673(93)83295-4
Gheysen, K., Mihai, C., Conrath, K., & Martins, J. C. (2008). Rapid identification of common hexapyranose monosaccharide units by a simple TOCSY matching approach. Chemistry - A European Journal, 14(29), 8869-8878. https://doi.org/10.1002/chem.200801081
Green, M. R., & Sambrook, J. (2012). Molecular Cloning: A laboratory manual. Fourth Editi*on.*
Gritz, L., & Davies, J. (1983). Plasmid-encoded hygromycin B resistance: the sequence of hygromycin B phosphotransferase gene and its expression in Escherichia coli and Saccharomyces cerevisiae. Gene, 25(2-3), 179-187. https://doi.org/10.1016/0378-1119(83)90223-8
Guilmanov, V., Ballistreri, A., Impallomeni, G., & Gross, R. A. (2002). Oxygen transfer rate and sophorose lipid production by Candida bombicola. Biotechnology and Bioengineering, 77(5), 489-494. https://doi.org/10.1002/bit.10177
Huang, F. C., Peter, A., & Schwab, W. (2014). Expression and characterization of CYP52 genes involved in the biosynthesis of sophorolipid and alkane metabolism from Starmerella bombicola. Applied and Environmental Microbiology, 80(2), 766-776. https://doi.org/10.1128/AEM.02886-13
Lang, S., Brakemeier, A., Heckmann, R., Spöckner, S., Rau, U., Spockner, S., & Rau, U. (2000). Production of native and modified sophorose lipids. Chimica Oggi - Chemistry Today, 18(10), 76-79.
Lodens, S., De Graeve, M., Roelants, S. L. K. W. L. K. W., De Maeseneire, S. L. L., & Soetaert, W. (2018). Transformation of an exotic yeast species into a platform organism: a case study for engineering glycolipid production in the yeast Starmerella bombicola. P. in J. Braman Ed. Synth. Biol. Springer Publishing Co. Series "Methods in Molecular Biology.," 1772, 95-123. https://doi.org/10.1007/978-1-4939-7795-6
Petersen, B. O., Vinogradov, E., Kay, W., Würtz, P., Nyberg, N. T., Duus, J., & Sørensen, O. W. (2006). H2BC: A new technique for NMR analysis of complex carbohydrates. Carbohydrate Research, 341(4), 550-556. https://doi.org/10.1016/j.carres.2005.11.020
Rau, U., Hammen, S., Heckmann, R., & Wray, V. (2001). Sophorolipids: a source for novel compounds. Industrial Crops and Products, 13, 85-92.
Roelants, S. L. K. W. K. W., Ciesielska, K., De Maeseneire, S. L., Moens, H., Everaert, B., Verweire, S., ... Soetaert, W. (2016). Towards the industrialization of new biosurfactants: Biotechnological opportunities for the lactone esterase gene from Starmerella bombicola. Biotechnology and Bioengineering, 113(3), 550-559. https://doi.org/10.1002/bit.25815
Roelants, S. L. K. W., Saerens, K. M. J., Derycke, T., Li, B., Lin, Y.-C., Van de Peer, Y., Soetaert, W. (2013). Candida bombicola as a platform organism for the production of tailor-made biomolecules. Biotechnology and Bioengineering, 110(9), 2494-2503. https://doi.org/10.1002/bit.24895
Saerens, K. M. J. (2012). Synthesis of glycolipids by Candida bombicola. Retrieved from http://lib.ugent.be/catalog/rug01:001796661
Saerens, K. M. J., Saey, L., & Soetaert, W. (2011). One-step production of unacetylated sophorolipids by an acetyltransferase negative Candida bombicola. Biotechnology and Bioengineering, 108(12), 2923-2931.
Soetaert, W., Van Bogaert, I., & Roelants, S. (2013). Methods to produce bolaamphiphilic glycolipids. WO 2015028278 A1. 50p.
Takahashi, F., Igarashi, K., & Hagihara, H. (2016). Identification of the fatty alcohol oxidase FAO1 from Starmerella bombicola and improved novel glycolipids production in an FAO1 knockout mutant. Applied Microbiology and Biotechnology, 100(22), 9519-9528. https://doi.org/10.1007/s00253-016-7702-6
Van Bogaert, I. N. A., Buyst, D., Martins, J. C., Roelants, S. L. K. W. K. W., & Soetaert, W. K. (2016). Synthesis of bolaform biosurfactants by an engineered Starmerella bombicola yeast. Biotechnology and Bioengineering, 113(12), 2644-2651. https://doi.org/10.1002/bit.26032
Van Bogaert, I. N. A., Holvoet, K., Roelants, S. L. K. W., Li, B., Lin, Y.-C. C., Van de Peer, Y., & Soetaert, W. (2013). The biosynthetic gene cluster for sophorolipids: a biotechnological interesting biosurfactant produced by Starmerella bombicola. Molecular Microbiology, 88(3), 501-509. https://doi.org/10.1111/mmi.12200
Van Bogaert, I. N., Roelants, S., Develter, D., & Soetaert, W. (2010). Sophorolipid production by Candida bombicola on oils with a special fatty acid composition and their consequences on cell viability. Biotechnology Letters, 32(10), 1509-1514. https://doi.org/10.1007/s10529-010-0323-8
Van Renterghem, L., Roelants, S. L. K. W., Baccile, N., Uyttersprot, K., Taelman, M. C., Everaert, B., ... Soetaert, W. (2018). From lab to market: An integrated bioprocess design approach for new-to-nature biosurfactants produced by Starmerella bombicola. Biotechnology and Bioengineering, 115(5), 1195-1206. https://doi.org/10.1002/bit.26539

## Claims

1. A method to produce fully symmetrical bola sophorosides which are free from contaminating fatty acid containing sophorolipids comprising feeding a mutated fungal strain with a fatty alcohol having an aliphatic tail chain length of at least 6 carbons wherein said fungal strain has a non-functional CYP52M1 cytochrome P450 monooxygenase and a non-functional FAO1 fatty alcohol oxidase and wherein said fungal strain is a yeast selected from the group consisting of *Starmerella (Candida) bombicola, Candida apicola, Candida batistae, Candida floricola, Candida riodocensis, Candida stellate, Candida kuoi, Candida sp. NRRL Y-27208, Rhodotorula bogoriensis sp., Wickerhamiella domericqiae* and a sophorolipid-producing strain of the *Starmerella* clade.

2. The method according to claim 1 wherein the gene encoding for the CYP52M1 cytochrome P450 monooxygenase and the gene encoding for the FAO1 fatty alcohol oxidase are knocked out.

3. The method according to claims 1-2 wherein said fatty alcohol having an aliphatic tail chain length of at least 6 carbons is hexanol, octanol, decanol, undecyl alcohol, lauryl alcohol, tridecyl alcohol, myristyl alcohol, pentadecyl alcohol, cetyl alcohol, palmitoleyl alcohol, heptadecyl alcohol, stearyl alcohol, isostearyl alcohol, oleyl alcohol, nonadecyl alcohol, arachidyl alcohol or a mixture thereof.

4. The method according to claims 1-3, wherein said symmetrical bola sophorosides are acetylated.

5. A mutated fungal strain having a non-functional CYP52M1 cytochrome P450 monooxygenase and a non-functional FAO1 fatty alcohol oxidase wherein said fungal strain is a yeast selected from the group consisting of *Starmerella (Candida) bombicola, Candida apicola, Candida batistae, Candida floricola, Candida riodocensis, Candida stellate, Candida kuoi, Candida sp. NRRL Y-27208, Rhodotorula bogoriensis sp., Wickerhamiella domericqiae* and a sophorolipid-producing strain of the *Starmerella* clade.

6. The mutated fungal strain according to claim 5 wherein the gene encoding for the CYP52M1 cytochrome P450 monooxygenase and the gene encoding for the FAO1 fatty alcohol oxidase are knocked out.

7. Use of a mutated fungal strain according to claims 5-6 to produce fully symmetrical bola sophorosides which are free from contaminating fatty acid containing sophorolipids such as acidic, lactonic or bola sophorolipids.

8. Use of a mutated fungal strain according to claims 5-6 to produce fully symmetrical terminal diols.

9. Use of a mutated fungal strain according to claim 7 wherein said symmetrical bola sophorosides are part of mixture further comprising alkyl sophorosides.

10. Use of a mutated fungal strain according to claims 5-6 to produce alkyl sophorosides.

11. The mutated fungal strain according to claim 5, wherein said fungal strain further comprises a non-functional glucosyltransferase UGTB1 that is responsible for the second glucosylation step in the sophorolipid biosynthetic pathway.

12. Use of mutated fungal strain according to claim 11 to produce symmetrical bola glucosides.

13. Use according to claim 12, wherein said symmetrical bola glucosides are acetylated.

## Patentansprüche

1. Verfahren zur Herstellung von vollsymmetrischen Bola-Sophorosiden, die frei von kontaminierenden fettsäurehaltigen Sophorolipiden sind, umfassend Füttern eines mutierten Pilzstamms mit einem Fettalkohol, der einer aliphatische Schwanzkettenlänge von mindestens 6 Kohlenstoffatomen aufweist, wobei der Pilzstamm eine nichtfunktionelle CYP52M1-Cytochrom-P450-Monooxygenase und eine nichtfunktionelle FAO1-Fettalkoholoxidase aufweist und wobei es sich bei dem Pilzstamm um eine Hefe handelt, die aus der Gruppe bestehend aus *Starmerella (Candida) bombicola, Candida apicola, Candida batistae, Candida floricola, Candida riodocensis, Candida stellate, Candida kuoi, Candida sp. NRRL Y-27208, Rhodotorula bogoriensis sp., Wickerhamiella domericqiae* und einem Sophorolipid produzierenden Stamm der *Starmerella*-Klade ausgewählt ist.

2. Verfahren nach Anspruch 1, wobei das Gen, das für die CYP52M1-Cytochrom-P450-Monooxygenase codiert, und das Gen, das für die FAO1-Fettalkoholoxidase codiert, durch Knock-out ausgeschaltet sind.

3. Verfahren nach Anspruch 1 - 2, wobei es sich bei dem einer aliphatische Schwanzkettenlänge von mindestens 6 Kohlenstoffatomen aufweisenden Fettalkohol um Hexanol, Octanol, Decanol, Undecylalkohol, Laurylalkohol, Tridecylalkohol, Myristylalkohol, Pentadecylalkohol, Cetylalkohol, Palmitoleylalkohol, Heptadecylalkohol, Stearylalkohol, Isostearylalkohol, Oleylalkohol, Nonadecylalkohol, Arachidylalkohol oder ein Gemisch davon handelt.

4. Verfahren nach Anspruch 1 bis 3, wobei die symmetrischen Bola-Sophoroside acetyliert sind.

5. Mutierter Pilzstamm, aufweisend eine nichtfunktionelle CYP52M1-Cytochrom-P450-Monooxygenase und eine nichtfunktionelle FAO1-Fettalkoholoxidase, wobei es sich bei dem Pilzstamm um eine Hefe handelt, die aus der Gruppe bestehend aus *Starmerella (Candida) bombicola, Candida apicola, Candida batistae, Candida floricola, Candida riodocensis, Candida stellate, Candida kuoi, Candida sp. NRRL Y-27208, Rhodotorula bogoriensis sp., Wickerhamiella domericqiae* und einem Sophorolipid produzierenden Stamm der *Starmerella*-Klade ausgewählt ist.

6. Mutierter Pilzstamm nach Anspruch 5, wobei das Gen, das für die CYP52M1-Cytochrom-P450-Monooxygenase codiert, und das Gen, das für die FAO1-Fettalkoholoxidase codiert, durch Knock-out ausgeschaltet sind.

7. Verwendung eines mutierten Pilzstamms nach Anspruch 5 - 6 zur Herstellung vollsymmetrischer Bola-Sophoroside, die frei von kontaminierenden fettsäurehaltigen Sophorolipiden wie sauren, Lacton- oder Bola-Sophorolipiden sind.

8. Verwendung eines mutierten Pilzstamms nach Anspruch 5 - 6 zur Herstellung vollsymmetrischer terminaler Diole.

9. Verwendung eines mutierten Pilzstamms nach Anspruch 7, wobei die symmetrischen Bola-Sophoroside Teil eines Gemischs sind, das ferner Alkylsophoroside umfasst.

10. Verwendung eines mutierten Pilzstamms nach Anspruch 5 - 6 zur Herstellung von Alkylsophorosiden.

11. Mutierter Pilzstamm nach Anspruch 5, wobei der Pilzstamm ferner eine nichtfunktionelle Glucosyltransferase UGTB1 umfasst, die für den zweiten Glucosylierungsschritt im Sophorolipid-Biosyntheseweg verantwortlich ist.

12. Verwendung des mutierten Pilzstamms nach Anspruch 11 zur Herstellung symmetrischer Bola-Glucoside.

13. Verwendung nach Anspruch 12, wobei die symmetrischen Bola-Glucoside acetyliert sind.

## Revendications

1. Procédé pour produire des sophorosides bola totalement symétriques qui sont exempts de sophorosides contaminants contenant des acides gras, comprenant l'alimentation d'une souche fongique mutée avec un alcool gras ayant une longueur de chaîne de queue aliphatique d'au moins 6 atomes de carbone, dans lequel ladite souche fongique a une cytochrome P450 monooxygénase CYP52M1 non fonctionnelle et une alcool gras oxydase FAO1 non fonctionnelle et dans lequel ladite souche fongique est une levure choisie dans le groupe constitué par Starmerella (Candida) bombicola, Candida apicola, Candida batistae, Candida floricola, Candida riodocensis, Candida stellate, Candida kuoi, Candida sp. NRRL Y-27208, Rhodotorula bogoriensis sp., Wickerhamiella domericqiae et une souche productrice de sophorolipides du clade Starmerella.

2. Procédé selon la revendication 1, dans lequel le gène codant pour la cytochrome P450 monooxygénase CYP52M1 et le gène codant pour l'alcool gras oxydase FAO1 sont inactivés par knock-out.

3. Procédé selon les revendications 1-2 dans lequel ledit alcool gras ayant une longueur de chaîne de queue aliphatique d'au moins 6 atomes de carbone est l'hexanol, l'octanol, le décanol, l'alcool undécylique, l'alcool laurylique, l'alcool tridécylique, l'alcool myristylique, l'alcool pentadécylique, l'alcool cétylique, l'alcool palmitoléylique, l'alcool heptadécylique, l'alcool stéarylique, l'alcool isostéarylique, l'alcool oléylique, l'alcool nonadécylique, l'alcool arachidylique ou un mélange de ceux-ci.

4. Procédé selon les revendications 1 à 3, dans lequel lesdits sophorosides bola symétriques sont acétylés.

5. Souche fongique mutée ayant une cytochrome P450 monooxygénase CYP52M1 non fonctionnelle et une alcool gras oxydase FAO1 non fonctionnelle, ladite souche fongique étant une levure choisie dans le groupe constitué par Starmerella (Candida) bombicola, Candida apicola, Candida batistae, Candida floricola, Candida riodocensis, Candida stellate, Candida kuoi, Candida sp. NRRL Y-27208, Rhodotorula bogoriensis sp., Wickerhamiella domericqiae et une souche productrice de sophorolipides du clade Starmerella.

6. Souche fongique mutée selon la revendication 5, dans laquelle le gène codant pour la cytochrome P450 monooxygénase CYP52M1 et le gène codant pour l'alcool gras oxydase FAO1 sont inactivés par knock-out.

7. Utilisation d'une souche fongique mutée selon les revendications 5-6 pour produire des sophorosides bola totalement symétriques qui sont exempts de sophorosides contaminants contenant des acides gras tels que des sophorolipides acides, lactoniques ou bola.

8. Utilisation d'une souche fongique mutée selon les revendications 5-6 pour produire des diols terminaux totalement symétriques.

9. Utilisation d'une souche fongique mutée selon la revendication 7, dans laquelle lesdits sophorosides bola symétriques font partie d'un mélange comprenant en outre des alkyl-sophorosides.

10. Utilisation d'une souche fongique mutée selon les revendications 5-6 pour produire des alkyl-sophorosides.

11. Souche fongique mutée selon la revendication 5, dans laquelle ladite souche fongique comprend en outre une glucosyltransférase non fonctionnelle UGTB1 qui est responsable de la seconde étape de glucosylation dans la voie biosynthétique de sophorolipides.

12. Utilisation d'une souche fongique mutée selon la revendication 11 pour produire des glucosides bola symétriques.

13. Utilisation selon la revendication 12, dans laquelle lesdits glucosides bola symétriques sont acétylés.
